# EUROPEAN PATENT SPECIFICATION

(11) **EP 1 556 682 B1**
(45) Date of publication and mention of the grant of the patent: **07.10.2009**
(21) Application number: 03809590.7
(22) Date of filing: 20.10.2003
(51) Int. Cl.: G01N 33/68, G01N 33/542, G01N 33/532

(54) **IP sb 3 /sb PROTEIN BINDING ASSAY**
IP3-PROTEIN-BINDUNGSTEST
BIODOSAGE DE IP sb 3 /sb PAR LIAISON DE PROTEINE

(30) Priority: 21.10.2002 US 420469 P
(43) Date of publication of application: 27.07.2005
(73) Proprietor: DiscoveRx Corporation, Fremont, CA 94538 (US)
(72) Inventor: NAQVI, Tabassum, Fremont, CA 94538 (US); ROUHANI, Riaz, Concord, CA 94521 (US); FUNG, Peter, Sunnyvale, CA 94086 (US); EGLEN, Richard, Los Altos, CA 94024 (US); SINGH, Rajendra, San Jose, CA 95135 (US)
(74) Representative: Sexton, Jane Helen
(86) International application number: PCT/US2003/033262
(87) International publication number: WO 2004/038369

(56) References cited:
- EP-A2- 0 992 587
- US-A- 5 252 707
- US-A- 5 977 311
- BOSSE R ET AL: "RAPID AND ULTRA-SENSITIVE DETECTION OF CAMP AND IP3 USING THE HOMOGENEOUS AND NON-RADIOACTIVE PLATFORM ALPHASCREEN" FASEB JOURNAL (FEDERATION OF AMERICAN SOCIETIES FOR EXPERIMENTAL BIOLOGY), BETHESDA, US, vol. 16, no. 4, 4 March 2002 (2002-03-04), page A575, XP009022719 ISSN: 0892-6638
- ROSSI F ET AL: "MONITORING PROTEIN-PROTEIN INTERACTIONS IN INTACT EUKARYOTIC CELLS BY BETA-GALACTOSIDASE COMPLEMENTATION" PROCEEDINGS OF THE NATIONAL ACADEMY OF SCIENCES OF USA, NATIONAL ACADEMY OF SCIENCE, WASHINGTON, DC, US, vol. 94, August 1997 (1997-08), pages 8405-8410, XP002064565 ISSN: 0027-8424
- DATABASE BIOSIS [Online] BIOSCIENCES INFORMATION SERVICE, PHILADELPHIA, PA, US; 1990, HIRATA M ET AL: "STEREOSPECIFIC RECOGNITION OF INOSITOL 1 4 5-TRISPHOSPHATE ANALOGS BY THE PHOSPHATASE KINASE AND BINDING PROTEINS" XP002411337 Database accession no. PREV199090036981 & JOURNAL OF BIOLOGICAL CHEMISTRY, vol. 265, no. 15, 1990, pages 8404-8407, ISSN: 0021-9258
- RILEY ANDREW M ET AL: "Interactions of inositol 1,4,5-trisphosphate (IP3) receptors with synthetic poly(ethylene glycol)-linked dimers of IP3 suggest close spacing of the IP3-binding sites." JOURNAL OF BIOLOGICAL CHEMISTRY, vol. 277, no. 43, 25 October 2002 (2002-10-25), pages 40290-40295, XP002411424 ISSN: 0021-9258
- PERKINELMER LIFE SCIENCES: "Performing AlphaScreen IP3 Functional Assay" 31 July 2002 (2002-07-31), , XP002412571 * pages 2-3 *

## Description

### BACKGROUND OF THE INVENTION

### FIELD OF THE INVENTION

The invention concerns the measurement of IP₃ (D-myo-inositol, 1,4,5 trisphosphate).

### BACKGROUND INFORMATION

IP₃ plays an essential role as a second messenger regulating cellular Ca⁺⁺ by controlling the release of calcium from calcium stores in the endoplasmic reticulum into the cytoplasm. The receptor for IP₃ is a gated calcium release channel residing at the calcium storage sites. IP₃ is one of a family of phosphorylated inositol compounds that play different roles. The inositol family of phosphate esters differ as to the number of phosphates, the position of the phosphates, as well as their stereochemistry, so as to include both geometric and stereochemical isomers. A family ofphosphatases and kinases provide for rapid interchange between the different inositol phosphates. Because of the importance of calcium levels in the cytoplasm, IP₃ is an analyte of great interest.

Measurement of intracellular second messengers such as cAMP or IP₃ has typically been used to decipher signaling events in the cell mediated through GPCRs. GPCRs constitute the largest subgroup (about 45%) of all the molecular targets that are currently being pursued in drug discovery programs. These receptors transduce the binding of extracellular ligands into intracellular signaling events that are mediated through guanine nucleotide binding regulatory proteins (G-proteins). Traditionally, drug discovery programs targeting GPCRs have relied on the use of tissue preparations from native sources to perform screens of medicinal and natural product libraries.

The number of similar inositol phosphates makes IP₃ a difficult target to analyze. Also, the simplicity of the molecule and its low antigenicity makes it difficult to generate high affinity antibodies to IP₃. Any modification of IP₃ changes the character of the molecule, so that in any competitive assay where a derivative must be used, the modification must not significantly change the affinity of the labeled derivative as compared to the naturally occurring EP₃. For the most part the assays for IP₃ have depended upon using radioactive tags where the labeled compound is chemically identical to the naturally occurring IP₃.

While radioactive isotopic assays have high sensitivity and provide a labeled analog that can successfully compete for proteins binding IP₃, there are many undesirable aspects about using radioactive isotopes as a label. The use of radioactivity is dangerous; has serious disposal problems and since the time of Berson and Yalow's discovery of radioimmunoassay, the diagnostic field has moved away from the use of radioactive labels, to such other labels as fluorescers, enzymes, particles, enzyme fragment complementation and the like. There is a substantial interest in developing assays that avoid the use of radioisotopes, while providing the necessary sensitivity and specificity for detecting IP₃, without interference from the other inositol phosphate congeners.

### DESCRIPTION OF RELEVANT LITERATURE

Derivatives of IP3 are described in Marecek, et al., Carbohydrate Res. 1992, 234, 65-73; Guo, et al., Bioorg & Biochem. 1994,2, 7-13; Liu and Potter, J. Org. Chem. 1997, 62, 8335-40; and Chen, et al., J. Org. Chem. 1996, 61, 393-7. Methods for analytical separation of inositol phosphates are illustrated in U.S. Patent no. 5,225,349 and Hamada, J. Chromatog. A, 2002, 944, 241-8. Radioactive protein binding assays are described in Anderson, et al., J. Chromatog. 1992, 574, 150-5; Hingorani and Agnew, Anal. Biochem. 1991, 194, 204-13; and Bredt, et al., Biochem. Biophys. Res. Commun. 1989,159, 976-82. Antibodies for IP₃ and the derivatives used for preparing the antibodies are described in Shieh and Chen, Biochem. J. 1995, 311, 1009-14; Chen and Chen, et al., U.S. Patent nos. 5,393,912 and 5,798,447 and PCT application serial no. WO95/19373. Binding proteins other than antibodies for IP₃ are described in U.S. Patent no. 6,087,483; EPA 0,992,587 and Uchimaya, et al., J. Biol. Chem. 2002, 277, 8106-113. U.S. Patent no. 5,252,707 describes the preparation of derivatives of IP₃. Packard Bioscience, Alpha Screen Technology, Application Note ASC-018, Homogeneous Inositol 1,4,5-Trisphosphate (IP₃) Functional Assay for the G_{q}-coupled AT1 Receptor, describes a homogeneous assay for IP₃. The use of fluorescence polarization in assays is described by Owicki, "Fluorescence Polarization and Anisotropy in High Throughput Screening: Perspectives and Primer", Journal of Biomolecular Screening 2000, 5, 297-306. Bosse *et al*. describes rapid ultrasensitive detection of cAMP and IP₃ using homogeneous and non-radioactive platform Alphascreen^{™} (FASEB Journal, 16(4), A575, 2002).

The presence of thiol groups in IP³R is described in Kaplin, et al., 1994 J Biol Chem 269, 28972-78.

References describing the use of pleckstrin homology (PH) proteins for binding to vicinal diphosphate inositols include Hamman, et al., J. Biomol. Screening 2002, 7, 45-55; Dowler et al., Biochem. J. 2000, 351, 19-31; and Lemmon and Ferguson, 2000, Biochem. J. 2000, 350 1-18. A fluorescent *in vitro* biosensor for IP₃ using a fluorophore labeled pleckstrin homology domain has been described by Morii et al., J. Am. Chem. Soc. 2002, 124, 1138-9.

### SUMMARY OF THE INVENTION

Sensitive and specific non-radioactive protein binding assays are provided using an IP₃ derivative labeled at the 2-position and a truncated EP₃ receptor protein. The label is a small molecule of less than about 10kD. The sample is processed to inactive phosphatases and kinases, combined with the above-indicated reagents and the amount of bound or unbound label determined. Enzyme fragment complementation and is used for detection.

### BRIEF DESCRIPTION OF THE FIGURES

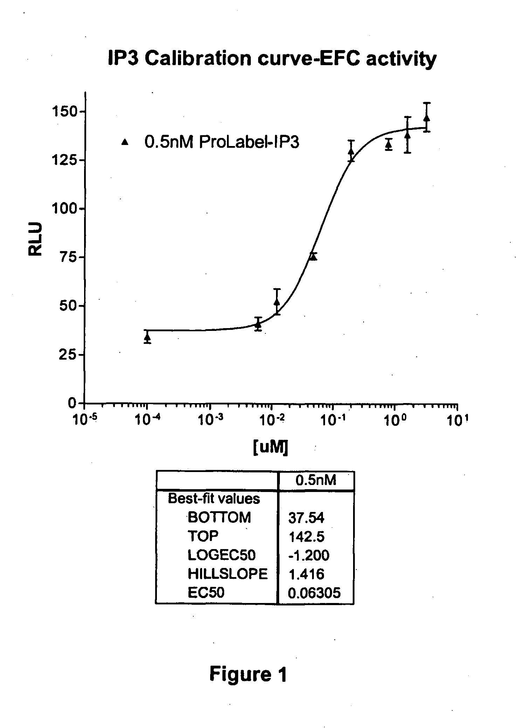

Fig. 1 is a calibration curve of an assay using enzyme fragment complementation to determine IP₃ concentration;

Fig. 2 is a graph of the effect of the addition of DTT to the binding protein buffer on stability of the assay;

Fig. 3a is a fluorescence polarization calibration curve using IP₃ binding protein in PD 10 buffer in the presence and absence of DTT;

Fig 3b shows the of the results of ligand induced IP₃ production from CHO-M1 cells measured by fluorescence polarization.

Fig. 4 is a table of results and a graph of the results using Cy3B fluorescer in an IP₃ fluorescence polarization assay;

Fig. 5 is a table of results and a graph of the results using hexachlorofluorescein fluorescer in an IP₃ fluorescence polarization assay;

Fig. 6 is a table of results using Alexa fluorescer in an IP₃ fluorescence polarization assay;

Fig. 7 is a graph of the results using fluorescence polarization and carbachol induction on an ATCC CHO-M1 cell line; and

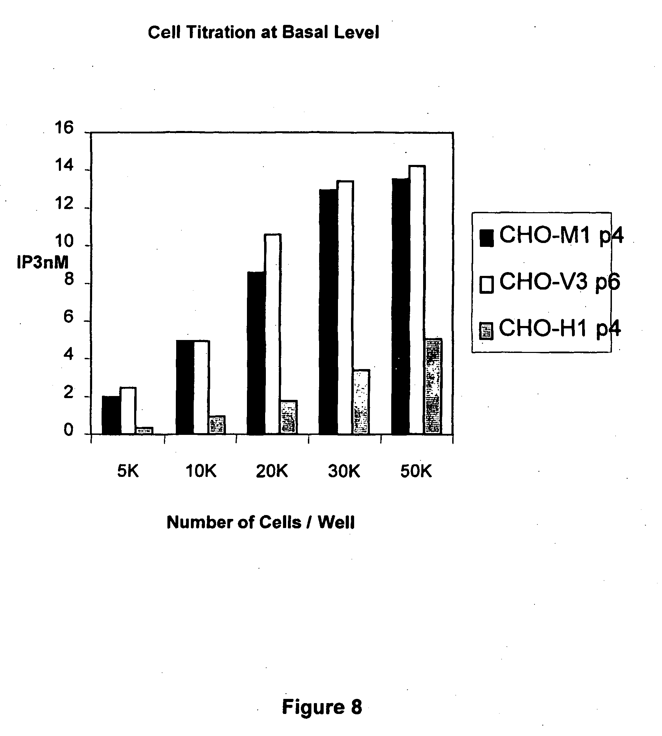

Fig. 8 is a bar graph of the determination of IP₃ at basal level with three different cell lines counting the number of cells.

### DESCRIPTION OF THE SPECIFIC EMBODIMENTS

In accordance with the subject invention non-radioactive protein-binding assays for IP₃ are provided. Cellular samples are processed to inactivate kinases and phosphatases to retain the naturally occurring IP₃ concentration. The sample may be further processed or modified, either before or after inactivation to prepare the sample for the assay. The processed sample is combined with a labeled IP₃, where the label is a derivative joined at the 2-position, particularly through an ether or ester group, usually through a linker. Depending upon the nature of the label, the molecular weight range will vary. For the enzyme label, the label will be less than about 30kD, usually less than about 10kD, preferably less than about 8kD. Therefore, the label will generally range in molecular weight from about 0.2kD to up to about 30kD.

A high affinity binding protein derived from an IP₃ receptor is employed as the binding protein. The sample and reagents are combined where the labeled derivative competes with the sample IP₃ for binding to the binding protein. Either or both the bound or unbound label may be determined. Of particular interest are homogeneous protein binding assays, which avoid a separation step after the combining of the sample and reagents.

In describing the invention, the reagents employed will be considered first. The labeled derivative or IP₃ analog will have a hydrogen bonded to oxygen at the IP₃ 2-position replaced, directly or usually through a linker to a detectable label, where the detectable label can provide a signal directly or indirectly, that is, additional reagents may be required. The linker includes the functionality, if any, to which the label is bonded and any functionality bonded to the 2-hydroxyl. The remaining portion of the linker other than the terminal functional groups will be referred to as the linking group, which may be a bond, but will usually be a chain. The chain will usually be of at least 2 atoms with a carbon atom bonded to the oxygen at the IP₃ 2-position, there being not more than about 16 atoms in the chain, usually not more than about 12 atoms in the chain (for a cyclic group the shortest link will be counted), where the atoms are carbon, nitrogen, oxygen, sulfur and phosphorous, where carbon atoms and heteroatoms may be in the chain or as substituents bonded to atoms in the chain. The linker will usually be at least one atom other than hydrogen and not more than about 30 atoms other than hydrogen, usually in the range of about 4 to 25 atoms. For the most part, the linker will be neutral or anionic, although cationic groups may be present, but will usually not be employed. The functional groups that are employed for the linker and the attaching functionality will be described below. The attaching functionality for the label will vary widely depending upon the nature of the label, where synthetic convenience, absence of interference with the assay, and high affinity of the derivative will direct the functionality that is employed.

The labeled derivative composition may not be a pure composition, generally having at least about 75% of the 2-position derivative, particularly at least about 90% and more particularly approaching at least 99%, preferably 100%, of the 2-position derivative. The cations are not considered as part of the derivative composition, since they will ionize in solution. For the most part the cations will be ammonium and alkali metal cations.

The labeled derivative will for the most part have the following formula:

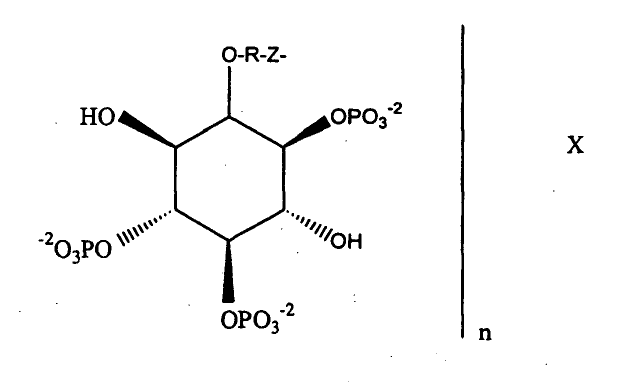

wherein:

R is a bond or linking group, usually a linking group of at least about 1 atom, usually at least about 2 atoms and more usually at least about 4 atoms, other than hydrogen, wherein said atoms include at least one carbon atom, there being not more than about 16 atoms in the chain, usually not more than about 12 atoms in the chain, which besides carbon atoms, may include the heteroatoms nitrogen, phosphorous, oxygen and sulfur, there generally being from 0 to 6, more usually 0 to 4 heteroatoms, more usually 1 to 4 heteroatoms, wherein the linking group may also include such heteroatoms as substituents on the chain, including oxo, amino, oxy, and thio. R may be aliphatic, alicycyclic, aromatic or heterocyclic, or combinations thereof, particularly aliphatic, branched chain or straight chain, saturated or unsaturated, having not more than about 2 sites of unsaturation, usually saturated. Usually, the linking group will be neutral or negatively charged, preferably neutral, having from 1 to 3, usually 1 to 2 heterogroups. The linking group may be hydrophilic or hydrophobic.

Z is a functionality bonded to R linking the label to R and may include oxy, amido, thio, succinimidyl, amino, ureido, ester, phospho, thiophospho, oxalo, etc., or combinations thereof, generally being of a total of from about 1 to 10 atoms, including carbon atoms and heteroatoms. Any functionality may be used for linking that does not interfere with the role of the reagent, the group being chosen because of synthetic convenience, stability and lack of detrimental effect;

X is the label, generally of from about 150Dal to about 30kD or optionally higher, usually not more than about 10kD, preferably not more than about 6kD, except when a surface or insoluble label, where the molecular weight may be indeterminate . The label may be varied widely being selected to provide the desired sensitivity for the assay, the absence of interference from the other reagents in the assay, the absence of interference of binding of the derivative to the binding protein, and having a reasonable protocol, generally avoiding a separation step after the combining of the sample with the reagents; and

n is an integer of from 1 to 2 depending upon the nature of the label, usually being 1 with a fluorescent label and 1 or 2 with an enzyme donor ("ED") label (to be subsequently described).

The group bonded to the 2-hydroxyl may be a saturated carbon atom or carboxyl, including thiocarbonyl, usually oxo-carbonyl. Depending upon the nature of the label, one or more IP₃'s may be bound to the label.

Any label that provides a signal sufficiently sensitive to detect the dynamic range of IP₃ without any interference with the assay can be employed. There is an enormous diversity of labels that may find use. According to the present invention the labels used are enzyme fragments in complementation assays, e.g. EDs from β-galactosidase, β-lactamase, ribonuclease, e.g., ribonuclease S, etc.

Labels of particular interest include enzyme complementation fragments, such as the enzyme donor (ED) fragment from β-galactosidase and β-lactamase, fluorescers, and chemiluminescers, particularly the enzyme complementation fragments and fluorescers. (The ED is commonly referred to in the literature as an enzyme donor, being the smaller fragment as compared to the EA, the enzyme acceptor.) One can achieve complementation by either having an ED labeled ligand complex with EA, or make fusion proteins of the ED and EA, with complementary binding agents that will naturally complex and bring the ED and EA together. Of particular interest is the use of fragment complementation enzyme donors as employed with a small ED fragment of β-galactosidase (the small ED is also referred to as Prolabel or PL). The enzyme donor will be at least about 36 amino acids and not more than about 95 amino acids, usually not more than about 75 amino acids. It is found that a relatively large polypeptide does not interfere with the binding of the IP₃ derivative to the binding protein.

For ED, the ED may have one or two functionalities for linking. Of particular interest is where the ED has one to two thiol groups, generally as cysteines proximal to or at the termini of the ED, which thiol groups may be added to an activated olefin to form a thioether.

Illustrative linking groups linked to the 2-hydroxyl oxygen include propylamidobutyl, propylamidophenyl, propyloxypropyl, butylureidohexyl, phenyl, butylureidophenyl, pentyl, propyl phosphate diester, butyryloxypentyl, dibutyl phosphate ester, N-(N'-ethyl-2-propylamido) butyrylamido, hexylthioethyl, hexylthiophenyl, methoxyacetyl, diethyleneoxy, etc.

Of particular interest are conjugates in assays dependent on a binding protein obtained by truncating the extracellular part of a IP₃R, Type 1, 2 or 3, where there is a substantial enhancement of binding to IP₃ over the natural receptor, usually at least about 200-fold enhancement, preferably at least about 500-fold enhancement, and even 1000-fold enhancement or greater.

A binding protein for IP₃ is specifically described in EPA 0 992 587 and Uchiyama, et al., 2002, *supra*. Other IP₃ binding proteins may also be used that are derived from IP₃ receptors, following the procedure employed in the cited references. By isolating the IP₃R or expressing only the extracellular portion of the IP₃R and mildly trypsinizing or using another relatively non-specific protease, large fragments of the extracellular portion can be obtained. These can be isolated using labeled IP₃, e.g. with a radioisotope or biotin (including biotin mimic), employing chromatography, panning, streptavidin bound to a surface, etc. The affinity for IP₃ may then be determined in accordance with conventional assays or according to this invention. Alternatively, one may use labeled core protein employed in the subject invention in competition with truncated fragments of the IP₃R for labeled IP₃ and determine the extent to which the labeled IP₃ binds to the core protein in the presence of the truncated IP₃R fragments. One may then isolate the gene for the IP₃R and by manipulation of the gene determine the minimum number of amino acids of the fragment that maximize the affinity. Methods for identifying such monomer sequence are amply described in the literature including the references cited herein.

The significant factor is that the core protein or "sponge" is readily available and for the purposes of this invention only one protein is required that has the requisite characteristics. The core protein is derived from mouse type 1 IP₃R1. The core protein is amino acids 226 - 578, although the naturally occurring N- and C- amino acids may be included, usually to provide a protein of not more than 1.5k amino acids, preferably not more than about 750 amino acids and more preferably not more than about 600 amino acids. The extension need not be the naturally occurring amino acids, and may total 1 - 500 amino acids, usually not more than about 1 to 300 amino acids. The additional amino acids may serve a variety of purposes, such as aiding in the purification of the core protein, aiding in the isolation of the complex between the core protein and an IP₃ derivative, causing steric inhibition of complementation with a peptide label to its cognate protein, or attachment to a surface or another molecule, where the surface may be a plate, a microtiter well wall, a particle, or the like.

In some instances one may make a fusion protein of the binding protein, where the fused polypeptide may serve a variety of functions, such as ease of purification, enhanced stability under the conditions of the assay, in the use of FRET assays, using GFP and like variants, etc. Generally the fused polypeptide will be less than about 1kD, usually less than about 0.6kD and more usually less than about 0.5kD. Among fusion proteins of the binding protein, a fusion with GST has found use.

The binding protein or sponge has a plurality of thiol groups, namely seven thiol groups. The binding protein is available as a fusion protein with glutathione sulfur transferase, which provides for a total of 11 cysteines. It is found that better results are obtained when including a reductant that inhibits disulfide formation, such as dithiothreitol, bis-imide mercaptoacetyl, mercaptoethylamine, bisulfite, β-mercaptoethanol, etc. The amount of the reductant will generally be in the range of about 1-100mM.

The IP₃ derivatives can be prepared using the procedures described in U.S. Patent no. 5, 252,707. Beginning with the 4,5-diphosphate inositol, the 3,4,5 and 6 positions may be selectively protected using an aralkyl halide, e.g. benzyl chloride, leaving the 1- and 2-hydroxyl groups unprotected. The 1-hydroxyl may then be selectively protected using silylation, followed by using the unprotected 2-hydroxyl for nucleophilic substitution on an acyl group or saturated alkyl group having a displaceable functionality, e.g. halide or pseudohalide. The 1-position may then be phosphorylated removing the silyl group and the protecting groups removed providing the 2-derivative of the IP₃.

The assays employed can be homogeneous or heterogeneous protein binding assays, where the analyte IP₃ competes with a labeled IP₃ analog for a binding protein specific for the analyte. In homogeneous assays the binding of the protein to the analog to form a complex results in a change in an observed signal. In heterogeneous assays, the complex of the binding protein and the analog is sequestered to a surface, a well wall, a particle, e.g. magnetic particle, or other surface, where the assay medium may be removed and the bound complex washed, so as to remove any analog from the surface. The presence of the analog on the surface may then be determined. Of particular interest are assays employing enzyme donors in an enzyme fragment complementation assay, more particularly the ED of β-galactosidase.

For the assay, depending upon whether one is performing an *in vitro* assay or wishes to do a cellular assay, one may wish to grow cells to partial confluence or confluence for use. Once the cells have been expanded, they may then be harvested for use. Due to the plethora of activities with which IP₃ is involved, a large variety of cells may be employed. The cells may be neuronal, heart, liver, kidney, leukocytes, spleen, skin, muscle, epidermal, endothelial, retinal, mesenchymal, etc. The cells may be naturally occurring, e.g. primary cells, cell lines, genetically modified cells, and may be from any eukaryote, e.g. mammal, such as human, mouse, lagomorpha, porcine, etc.

Depending upon the purpose of the assay, the cells may be subject to prior treatment or used directly. For example, primary cells may be checked to determine their IP₃ content to evaluate the state of the cells. In other situations, one may be interested in the effect of an agent on IP₃ formation, degradation or modification. The agent will usually, but not necessarily, be a drug that is being screened as to its activity, either direct activity on the level of IP₃ or whether the drug has as a side effect an activity affecting the level of IP₃. Where the effect of a change of environment, e.g. presence of a drug, is being determined, the cells will usually be incubated in an appropriate nutrient medium for a period of at least about 5min and not more than about 6h. The number of cells required for the assay will usually be in the range of about 10² to 10⁷ more usually in the range of about 10³ to 10⁵. The concentration of IP₃ to be determined will generally be in the range of about 0.1 to 10nmolar, which is generally about the physiological concentration.

The cells are lysed. Prior to or subsequent to lysing the action of phosphatases or kinases is inhibited to prevent modifications of the inositol phosphates present in the cell. Blocking the enzymatic reaction may be achieved in a variety of ways. Heat may be employed using a pulse of at least about 60-80°C for a time in the range of about 0.25 to 120sec followed by rapid cooling. Alternatively, one may use pH, by using a strong acid at a concentration in the range of about 0.1 to 0.25% with the sample, either before or after adding the binding protein and the IP₃ derivative. Acids that find use include perchloric acid, trichloroacetic acid, trifluoroacetic acid, etc. Depending on the nature of the label, it may be necessary to neutralize the acid by using an appropriate base. The sample may be brought to about pH 6.5 to 8. After inactivation, debris and other large components, e.g. organelles, may be removed by centrifugation and the supernatant isolated. Alternatively, the sample may be aspirated.

Sample preparation may follow the procedures described in BIOTRAK cellular communication assays, D-myo-Inositol 1,4,5-trisphosphate (IP₃) [³H] assay system, code TRK 1000, Amersham Pharmacia Biotech. Alternatively, one may follow the procedure described in U.S. Patent no. 6,183,974, excluding the use of the radioactive myo-inositol. The procedure generally involves seeding cells into wells at a density of about 10⁵ - 10⁶, culturing for 2 or more days, incubating with a test compound in assay medium at 37°C at predetermined time periods, and arresting the cellular activity by aspiration and addition of ice-cold 5% TCA. The pH is then adjusted to 7.4 with conc. NaOH and tris base.

The volume of the sample per 100µl will generally be in the range of about 5 to 50µl while the concentration of the other reagents will depend upon the nature of the label and will follow the methodology for a particular label.

As illustrative of a particular protocol using the ED of β-galactosidase, the analog is the ED of β-galactosidase linked at the 2-hydroxyl of IP₃. Usually, the ED will have from 37 amino acids to about 90 amino acids, more usually up to about 60 amino acids, preferably not greater than about 56 amino acids. The binding protein is a truncated extracellular portion of an EP₃R, particularly the core protein described by Uchiyama, 2002, *supra*, from mouse IP₃R1, including amino acids 200 to 610, more particularly 226 to 578. After blocking the phosphate related enzymes, and modifying the sample as appropriate, e.g centrifugation, a volume of 5 - 50µl of the sample is combined with a volume of about 5 - 50µl of the binding protein to provide a total concentration in the final assay mixture in the range of about 0.1nM to 1µM more usually in the range of about 1 to 100nM. The mixture is then incubated, conveniently at room temperature for at least 1min, usually at least about 5min and not more than about 30min, there being no advantage in unduly extending the incubation period. At the end of the first incubation, about 5 - 50µl of the analog is added with the ED joined to the IP₃ at the 2-hydroxyl by a linking group including the attaching functionalities, generally of from 4 to 20 carbon atoms, where the final concentration of the analog in the assay medium will usually be in the range of about 10pM to 100nM, more usually in the range of about 0.1nM to 10nM. The mixture is then incubated for the period as described above for the first incubation.

After the second incubation EA is added in a volume of about 5 to 50µl and the mixture incubated for at least about 5min, usually at least about 10min and not more than about 60min, usually not more than about 45min. Generally the amount of EA will be at least equal to the concentration of the ED, usually in excess, generally not more than about 10-fold excess, more usually not more than about 5-fold excess. At this time about 5 to 50µl of a substrate providing a detectable signal is added, where the substrate is in substantial excess of the amount that will be turned over in the assay. Illustrative substrates, many of which are commercially available, include dyes and fluorescers, such as X-gal, CPRG, 4-methylumbelliferyl β-galactoside, resorufin β-galactoside, Galacton Star (Tropix, Applied Biosystems). The procedure follows the conventional procedure for other analytes described in the scientific and patent literature. See, for example, U.S. Patent nos. 4,708,929 and 5,120,653, as illustrative. The assay mixture may then be read at a specific time, e.g. 1 - 10min, or as a rate, taking readings at specific intervals. With a chemiluminescent readout, the signal may be integrated for a time period of from 0.1 s to 1min.

To further enhance sensitivity, one may add antibodies to the binding protein to further enhance the bulk around the ED. Antibodies can be antisera or monoclonal, preferably monoclonal. The antibodies would be added after incubation with the binding protein and the sample and IP₃ analog. The antibodies will generally be in a mole ratio of at least about 1:1 to the core protein and generally at least about 2:1, where there may be used antisera that binds to a plurality of epitopic sites on the core protein or one or more monoclonal antibodies where the different antibodies bind to different sites on the binding protein. The incubation with the antibodies can be in the time range of the other incubations.

Another protocol of specific interest (but not of the invention) is fluorescence polarization, where the label is a fluorescer. The methodology is well established and has been described in numerous patents, including devices for measurement, such as U.S. Patent nos. 6,455,861; 6,159,750; and 4,952,691. In performing the method, a sample suspected of containing IP₃ is mixed with the binding protein. By maintaining the concentration of the analog and binding protein and the ratio of analog and binding protein, the ratio of IP₃ complex to analog complex is directly proportional to the amount of IP₃ in the sample. Upon exciting the assay mixture with fluorescent light, particularly at or about the absorption maximum of the fluorescer, and measuring the polarization fluorescence emitted by the fluorescer, one is able to quantitatively determine the amount of IP₃ in the sample. The assay may be performed in any convenient buffer, e.g. borate, phosphate, tris, etc., at a temperature in the range of about 15 to 40°C, using some of the principles described above, such as order of addition and incubation.

The indicated specific assays have many advantages. They are highly specific, not subject to interference from other inositol phosphates, rapid, can be automated and have high sensitivity. As shown in the experimental section, the ED assay has a dynamic range of from 1.0 to 10³nM. Sensitivity increases with diminishing concentration of IP₃.

For convenience, the reagents can be provided in kits. Depending upon the specific label different components may be included. Each of the kits will include the IP₃ conjugate, and the binding protein as described previously, and may include instructions for performing the assay, particularly electronically encoded or written instructions, buffer, etc. For the enzyme fragment conjugate, there will also be included the enzyme acceptor fragment and substrate for the holoenzyme. Also included is a reductant, conveniently a thiol, more particularly a polythiol, such as dithiothreitol.

### EXPERIMENTAL

Example 1.

**A. Preparation of the D-myo-inositol-2-O-(2-(3-maleimidopropionyl) aminoethyl) -4,5-triphosphate (mp-2-O-ae-1,4,5-IP3).**

D-myo-inositol-2-O-(2-aminoethyl)-1,4,5-triphosphate (2-O-ae-1, 4, 5-IP3) was prepared according to a published procedure. Riley and Potter, Chem Commun, 2000, 983-984. To a solution of 2-O-ae-1,4,5-IP3 (1 mg) in sodium phosphate (100 mM, pH 8.0, 1 mL) was added 100 µL of dry acetonitrile. Succinimidyl-3-maleimidopropionate (3 mg) was dissolved in minimum of acetonitrile (∼ 200 µL). The maleimide solution was slowly added to the amine solution and the reactants mixed by vortexing. The mixture was allowed to stand for 10 minutes. The product was isolated by HPLC and identified by FAB mass spectroscopy.

**B. Preparation of the PL47mdiCys conjugate of D-myo-inositol-1-(3-(3-maleimidopropionyl) aminopropyloxy)-4,5-triphosphate (PL47m-(mp₋1P-ap-1,4,5-IP3)₂).**

To a solution of freshly desalted PL47mdiCys (∼ 0.5 mg, 93 nmoles) in sodium phosphate (100 mM , pH 7.0) was added mp-2-O-ae-1,4,5-IP3 (0.35 mg, 557 nmoles) in water. (PL47mdiCys is amino acids 4 to 51 of E. coli β-galactosidase with cysteines added at both termini.) The mixture was reacted for 60 min. The product was purified by preparative HPLC using a gradient of 100 mM triethyl ammonium acetate (pH 7.0) and acetonitrile. Fractions containing the conjugate were identified by MALDI-TOF mass spectroscopy.

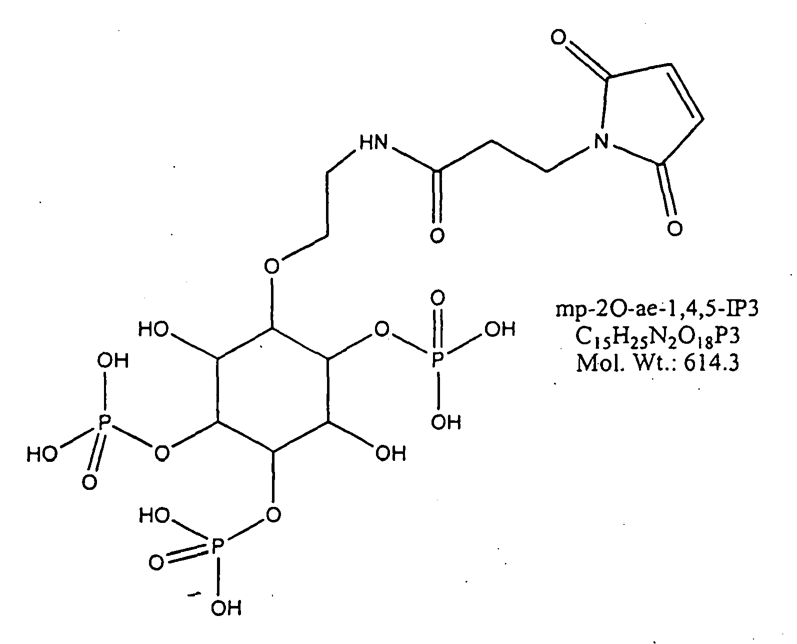

Preparation of the 2-O-(2-aminoethyl-(6-carboxamidofluoresceinyl))-D-myo-inositol-1, 4, 5-triphosphoric acid (not used in the claimed invention)

2-O-(2-aminoethyl)-D-myo-inositol-1,4,5-triphosphoric acid is prepared by the method of Riley and Potter, Chem. Comm., 983-984, 2000. To a solution of 2-O-(2-aminoethyl)-D-myo-inositol-1,4,5-triphosphoric acid (1 mg) in sodium phosphate (100 mM, pH 8.0, 0.5 mL) is added 100 µL of dry acetonitrile. Succinimidyl 6-carboxyfluorescein (1 mg) is dissolved in a minimum of dry DMF (∼ 100 µL). The activated ester of fluorescein solution is slowly added to the inositol solution, and the reactants mixed by vortex action. The mixture is allowed to stand for 60 minutes to complete the reaction. The product is isolated by HPLC and identified by mass spectroscopy.

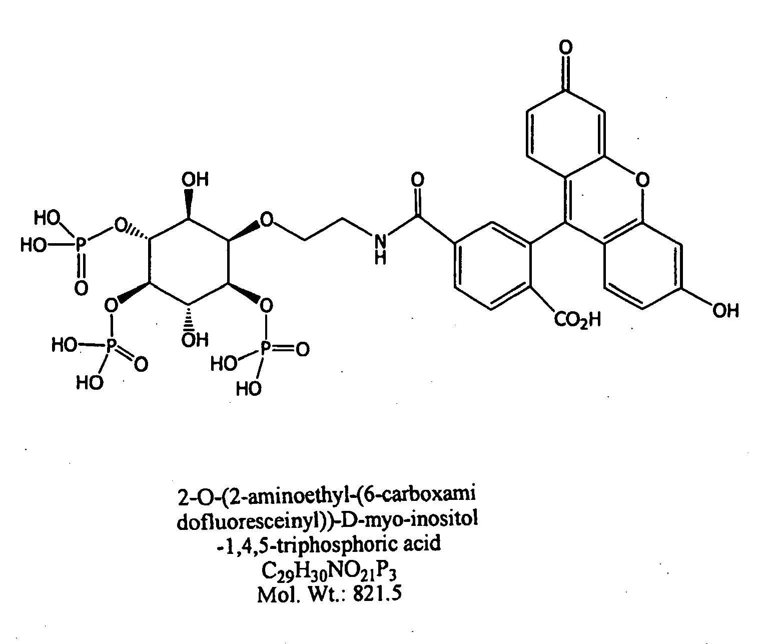

The other fluorescers were conjugated in substantially the same way. To a solution of 2-O-(2'-aminoethyl)-D-myo-inositol-1,4,5-triphosphoric acid triethylammonium salt (0.5mg) in 0.5ml of HPLC grade water was added 100 µL of dry acetonitrile. The N-hydroxy succinimide ester of the dye (0.5mg AlexaFluor 532 and hexachlorofluorescein, Molecular Probes, Eugene, OR; Cy3B, Amersham Biosciences, Buckinghamshire, UK) was dissolved in 100µl of dry DMF. The dye solution was added to the IP₃ solution while stirring and the reaction was allowed to proceed overnight at room temperature. The product was purified by HPLC on a reverse phase column (C18) with a triethylammonium acetate (100mM, pH 7.0) water/acetonitrile gradient.

Example 2

IP3 Binding buffers: Buffer A:50 mM Tris, pH 8.0, 1 mM β-mercaptoethanol, 1 mM EDTA, + 1X Complete Protease inhibitor cocktail (from Roche); The IP3 calibrator is resuspended in Buffer A at a stock concentration of 10 mM and then diluted in Buffer A to the various concentrations tested in the assay; The recombinantly expressed IP3 core binding domain protein is diluted in Buffer A (1:150 dilution).

Steps in the assay to generate the calibration curve: Using the IP3 core binding protein, the assay is done in a 384 well white Packard plate. Each reaction that makes up the calibration curve is performed in triplicate. The following is the order of steps of the assay:
1. Pipet 10 µl of IP3 calibrator into the well. The calibrator is titrated from a high concentration of 138 µM to 0.007 µM [final concentration].
2. Add 15 µl of the IP3 binding protein [diluted to a concentration of 0.01 µg/ µl] to the well and incubate for 10 minutes at room temperature.
3. Add 10 µl of the ProLabel-IP3 conjugate (0.5nM reagent concentration) to the well and incubate for 10 minutes at room temperature.
4. Add 10 µl of 0.1X EA to the well and incubate for 30 minutes at room temperature.
5. Add 20 µl of Chemiluminescent substrate (2X reagent concentration) to each of the wells. The plate is incubated at room temperature until ready to read.
Read the plate/samples after 15 minutes incubation with the substrate. Additional readings are taken after 30, 60 and 120 minutes. Samples are read on the Packard Lumi-count, with a PMT=1100 and Gain=1.

Analysis of data:

Samples are corrected against background activity in the binding protein samples (ie., RLUs are measured when the binding protein and buffer is incubated with the chemiluminescent substrate). After the background is subtracted from each triplicate sample, the replicates are averaged. The % Inhibition ("Open reading" {ProLabel-IP3 + EA + substrate} -"Close reading" {ProLabel-IP3 + IP3 binding protein + EA + Substrate} divided by the "Open reading" and times 100) and % Modulation (RLUs of the Calibrator tested -RLUs of the low calibrator divided by the calibrator tested times 100) are calculated and the data is imported into Prism Graphpad to generate a curve and determine the EC₅₀ of the binding reaction.

The signal to noise ratio is determined by the ratio between the highest calibrator divided by the lowest calibrator.

For the fluorescent polarization assays (not of the claimed invention), the following protocol was employed using DTT in the binding protein ("BP") buffer.

IP3 binding protein (at a concentration of 0.5µg/µl or 7.1 µM is diluted from 1:100 to 1:400 to provide a range of 72 to 18n) is diluted 1:300 in IP3 binding protein dilution buffer (BP dilution buffer-10 mM HEPES, 88 mM NaCl, 1 mM KCI, 0.1% BGG, 0.02% Tween-20, 25 mM DTT, pH7.4). A 1M stock of DTT is prepared and then diluted 1:40 into the BP dilution buffer base to make the IP3 BP dilution buffer. 10µL of calibrator or cells is added to the wells of a 96 well microtiter plate, with 5µL of ligand (inducing agent) or water and 5µL of 0.2N perchloric acid. To the above solution is added 10µL of the IP3-fluorescent derivative in 500mM TABS, pH9. After incubating for 5min, 20µL of the BP reagent solution is added and the plate read in a fluorescence polarization reader.

When performing the assay with cells, the protocol was modified as follows. CHO-M1 cells were obtained from Euroscreen (Brussels, Belgium) Cells were grown in F12 Media, 10% FBS, 1X Glu, 500 µg/mL G418. Cells were plated at 2x10⁵ per well. Inductions were done using carbachol or acetylcholine (either at 1mM final concentration) or a titration of the concentration. Inductions were carried out for 20 seconds before the addition of 0.2N PCA. Additional stable cell lines expressing the Histamine 1 receptor or the Vasopressin 3 receptor were also tested. Inductions were done using Histamine (titrating the concentration). The effects on basal levels of IP₃ detected were tested when the cell number was titrated. The basal levels of IP₃ detection increase with the cell number. Three different cells lines expressing different levels of transfected receptor were tested using both the IP₃ Green and Red Tracer ( the green tracer is fluorescein, while the red tracer is Alexa (532nm) fluorescer) assays. The assay is able to detect IP3 in cell lines with varying amounts of IP₃ receptor.

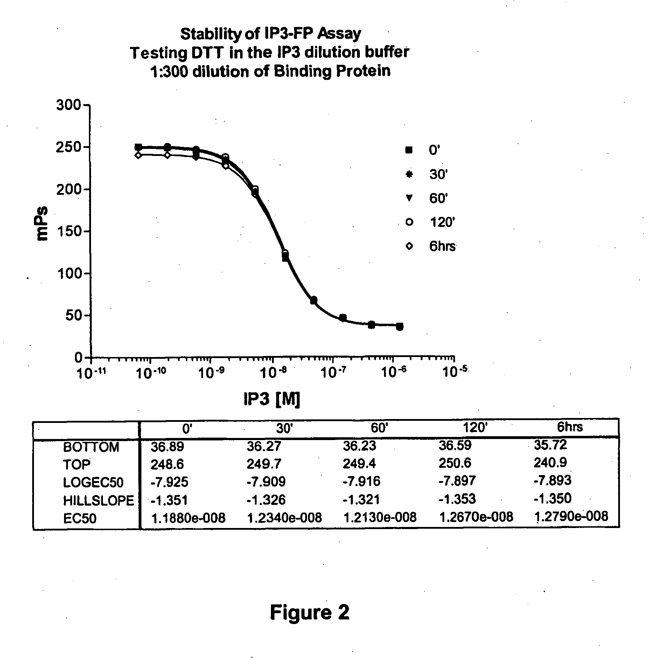

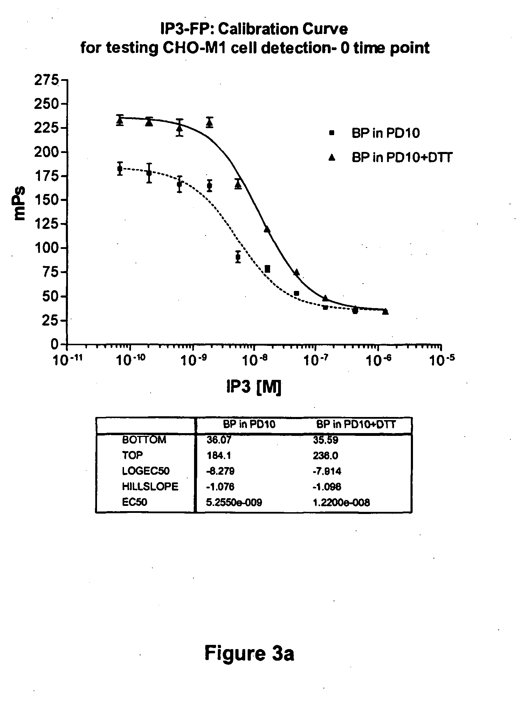

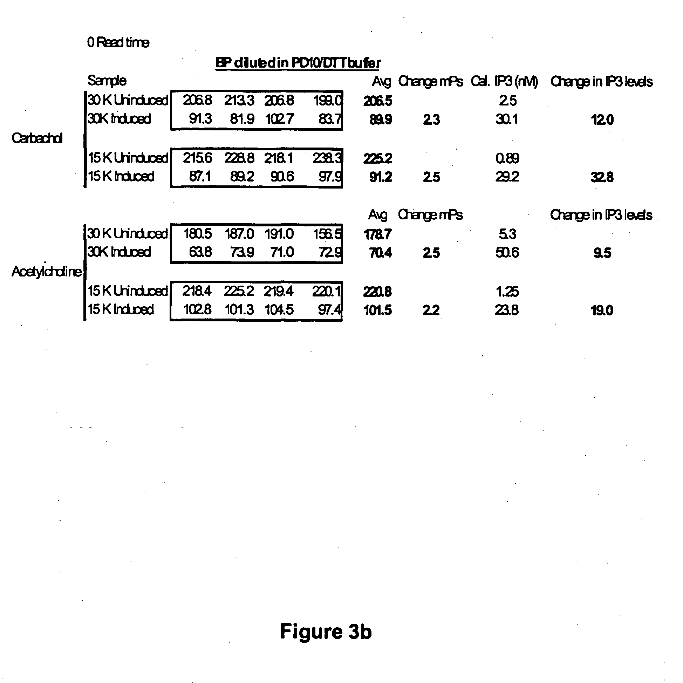

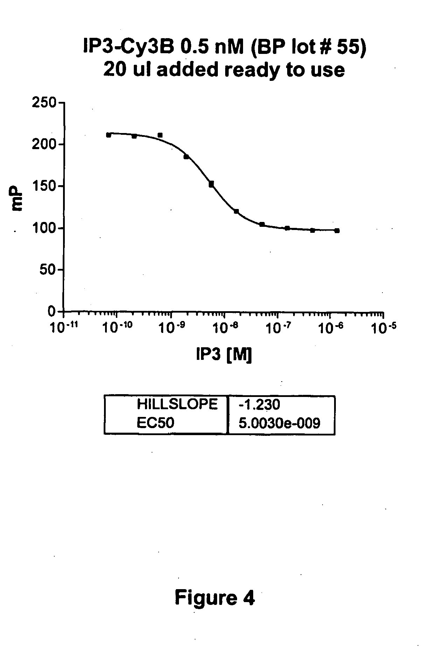

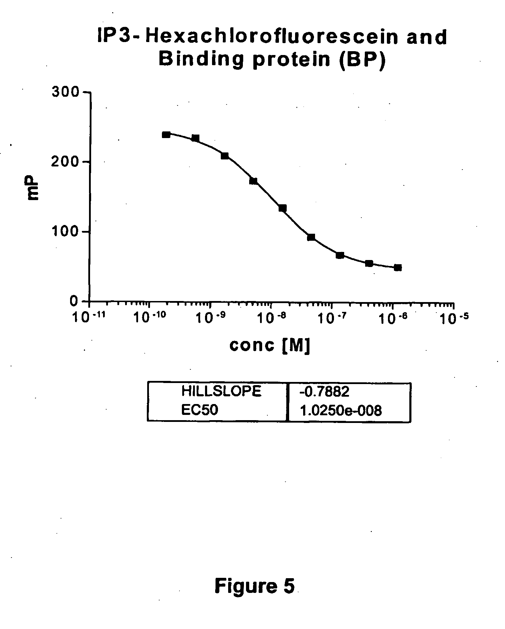

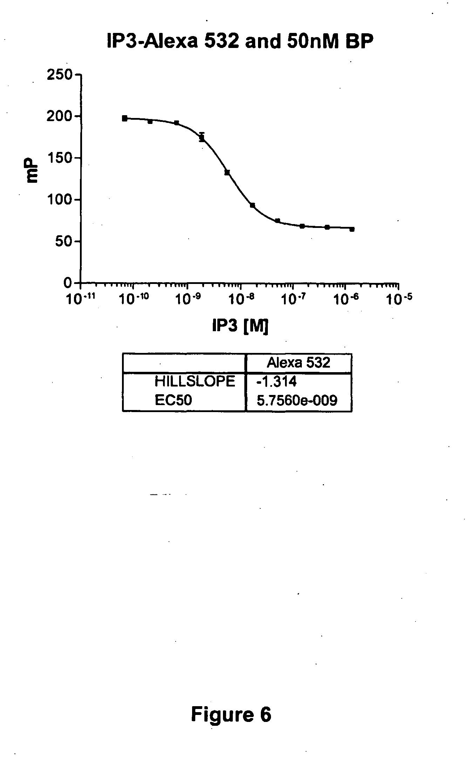

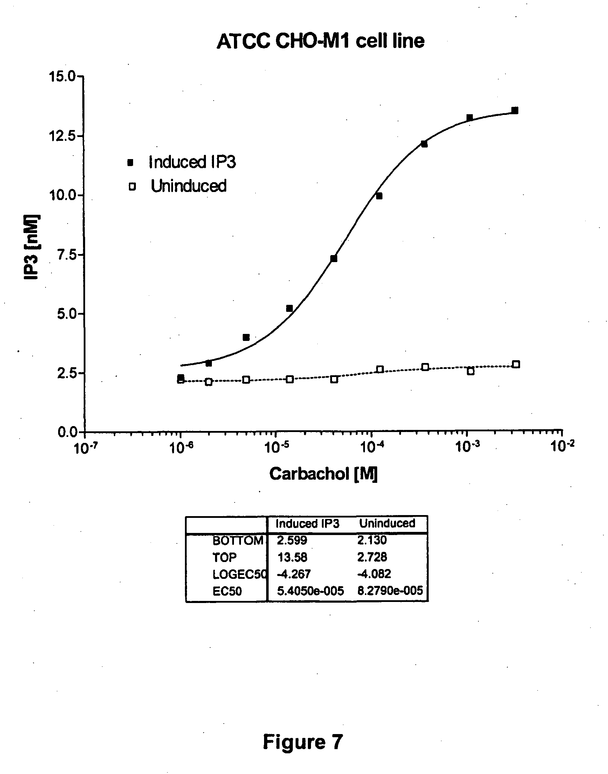

As shown in Figures 2 - 7, using the above protocol, accurate determination of the amount of IP-3 is readily determined over a wide dynamic range with different cell lines, under different conditions of induction.

The subject assays, particularly the homogeneous assays, have many virtues. They are simple to perform and can be readily automated and the results read with currently available equipment. They do not require the tedious separation steps and washings of heterogeneous assays that can introduce technician errors or other errors, even when automated. The assays are specific for IP₃, so that interference from other inositol phosphates is not encountered. The analog of IP₃ competes effectively for the binding protein with IP₃ to provide both sensitivity and specificity.

## Claims

1. A protein binding assay method for measuring D-myo-inosito1,1,4,5 triphosphate (IP₃) in a sample employing as reagents a conjugate of IP₃ and an enzyme fragment for enzyme complementation as a detectable label joined through a bond or linker at the 2-hydroxyl position, and a binding protein which is a truncated extracellular portion of an IP₃ receptor (IP₃R) having at least 200 times the affinity for IP₃ than the intact IP₃R, said method comprising:
combining in an assay medium said sample, said conjugate and said binding protein and incubating said mixture for sufficient time for any IP₃ and said conjugate to bind to said binding protein; and
detecting the bound or unbound label as a measure of the IP₃ present in the sample.

2. A protein binding assay according to claim 1, wherein said assay is in a homogeneous format.

3. A protein binding assay according to claim 1, wherein said sample is a cellular lysate, and wherein said cellular lysate has been treated to block kinases and phosphatases and prepare said sample for said assay.

4. A protein binding assay according to claim 1, wherein said binding protein is of not more than about 600 amino acids and comprises at least amino acids 226-578 of the mouse IP₃R Type 1.

5. A protein binding assay according to claim 1, wherein said binding protein is a fusion protein of up to 1.5k amino acids.

6. A method according to claim 1, wherein the order of addition of reagents is: (a) combining said sample with said binding protein; and (b) adding said conjugate, with incubating after (a) and (b).

7. A protein binding assay method for measuring EP₃ in a sample using a homogeneous format, employing as reagents a conjugate of IP₃ and an enzyme donor (ED) of from 37 to 60 amino acids derived from β-galactosidase joined through a linker at the 2-hydroxyl position, and a truncated extracellular portion of an IP₃R having at least about 200 times the affinity for EP₃ than the intact IP₃R, said method comprising:
combining in an assay medium assay components in the following order: said sample, said binding protein, said conjugate and enzyme acceptor (EA), and incubating after each combining for sufficient time for complex formation between said assay components;
adding substrate for said β-galactosidase; and
detecting the turnover of said β-galactosidase of said substrate as a measure of the IP₃ present in the sample.

8. A protein binding assay method for measuring IP₃ in a sample employing as reagents a conjugate of IP₃ and an enzyme fragment for enzyme complementation as a detectable label joined through a bond or linker at the 2-hydroxyl position, and a truncated extracellular portion of an IP₃R having at least about 200 times the affinity for IP₃ than the intact IP₃R, said method comprising:
combining in an assay medium said sample, said conjugate, said binding protein and a chemical reductant and incubating said mixture for sufficient time for any EP₃ and said conjugate to bind to said binding protein; and
detecting the bound or unbound label as a measure of the EP₃ present in the sample.

9. A protein binding assay according to claim 8, wherein said chemical reductant is a thiol.

10. A compound of the formula: 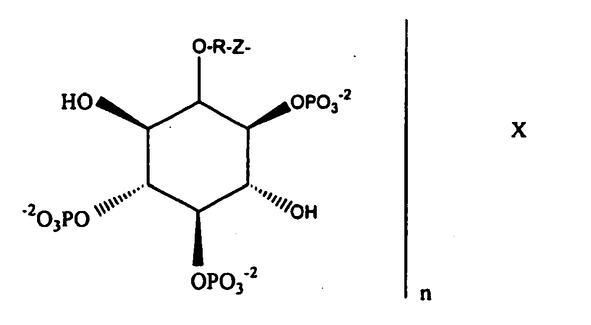 wherein:
R is a neutral linking group of from 4 to 20 carbon atoms bonded to the oxygen through a saturated carbon atom or carbonyl;
Z is a functionality for linking X to the oxygen at the 2-position;
X is an enzyme donor fragment of β-galactosidase of from 27 to 60 amino acids; and
n is 1 or 2.

11. A kit comprising a compound according to claim 10, enzyme acceptor for said enzyme donor and a truncated extracellular portion of an IP₃R having at least about 200 times the affinity for EP₃ than the intact IP₃R.

## Patentansprüche

1. Protein-Bindungsassayverfahren zur Messung von D-myo-inositol,1,4,5 triphosphat (IP₃) in einer Probe, die als Reagenz ein Konjugat aus IP₃ und einem Enzymfragment für die Enzymkomplementation als eine nachweisbare Markierung verwendet, die durch eine Bindung oder Verknüpfung an der 2-Hydroxyl Position verbunden sind, und einem Bindungsprotein, das ein gekürzter extrazellulärer Teil eines IP₃ Rezeptors (IP₃R) ist mit mindestens der 200-fachen Affinität für IP₃ als das intakte IP₃R, das genannte Verfahren umfasst:
Kombinieren der genannten Probe, des genannten Konjugats und des genannten Bindungsproteins in einem Assaymedium und Inkubieren der genannten Mischung für eine Zeit die ausreicht für jedes IP₃ und das genannte Konjugat sich an das genannte Bindungsprotein zu binden; und
Nachweisen der gebundenen oder nicht gebundenen Markierung als Maß für in der Probe vorhandenes IP₃.

2. Protein-Bindungsassay nach Anspruch 1, wobei der genannte Assay in einem homogenen Format ist.

3. Protein-Bindungsassay nach Anspruch 1, wobei die genannte Probe ein zelluläres Lysat ist, und wobei das genannte zelluläre Lysat behandelt wurde um Kinasen und Phosphatasen zu blockieren und Vorbereiten der genannten Probe für den genannten Assay.

4. Protein-Bindungsassay nach Anspruch 1, wobei das genannte Bindungsprotein aus nicht mehr als 600 Aminosäuren besteht und mindestens 226-578 Aminosäuren des Maus IP₃R Typ 1 umfasst.

5. Protein-Bindungsassay nach Anspruch 1, wobei das genannte Bindungsprotein ein Fusionsprotein aus bis zu 1,5k Aminosäuren ist.

6. Verfahren nach Anspruch 1, wobei die Reihenfolge der Zugabe der Reagenzien ist: (a) Kombinieren der genannten Probe mit dem genannten Bindungsprotein; und (b) Zugeben des genannten Konjugats, mit Inkubieren nach (a) und (b).

7. Protein-Bindungsassayverfahren zur Messung von IP₃ in einer Probe unter Einsatz eines homogenen Formats, das als Reagenz ein Konjugat aus IP₃ und einem Enzymdonor (ED) aus von β-Galactosidase abgeleiteten 37 bis 60 Aminosäuren verwendet, die durch Verknüpfung an der 2-Hydroxyl Position verbunden sind, und einem gekürzten extrazellulären Teil eines IP₃R mit mindestens der etwa 200-fachen Affinität für IP₃ als intaktes IP₃R, das genannte Verfahren umfasst:
Kombinieren von Assaykomponenten in einem Assaymedium in der folgenden Reihenfolge: die genannte Probe, das genannte Bindungsprotein, das genannte Konjugat und Enzymakzeptor (EA), und Inkubieren nach jeder Kombination für eine Zeit die ausreicht für die Komplexbildung zwischen den genannten Assaykomponenten;
Substratzugeben für die genannte β-Galactosidase; und
Nachweisen des Umsatzes der genannten β-Galactosidase des genannten Substrats als ein Maß für in der Probe vorhandenes IP₃.

8. Protein-Bindungsassayverfahren zur Messung von IP₃ in einer Probe, die als Reagenz ein Konjugat aus IP₃ und einem Enzymfragment für die Enzymkomplementation als eine nachweisbare Markierung verwendet, die durch eine Bindung oder Verknüpfung an der 2-Hydroxyl Position verbunden sind, und einem gekürzten extrazellulären Teil eines IP₃R mit mindestens der etwa 200-fachen Affinität für IP₃ als das intakte IP₃R, das genannte Verfahren umfasst:
Kombinieren der genannten Probe, des genannten Konjugats, des genannten Proteins und einem chemischen Reduktionsmittel in einem Assaymedium und Inkubieren der genannten Mischung für eine Zeit die ausreicht für jedes IP₃ und das genannte Konjugat an das genannte Bindungsprotein zu binden; und
Nachweisen der gebundenen oder nicht gebundenen Markierung als ein Maß für in der Probe vorhandenes IP₃.

9. Protein-Bindungsassay nach Anspruch 8, wobei das genannte chemische Reduktionsmittel ein Thiol ist.

10. Eine Verbindung der Formel: 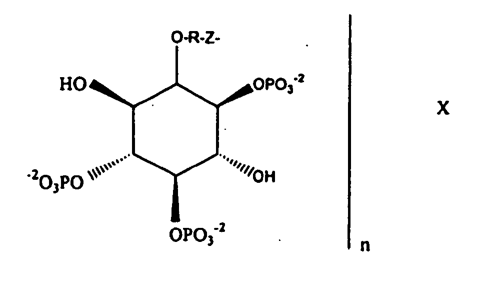 wobei:
R ist eine neutrale Verknüpfungsgruppe aus 4 bis 20 Kohlenstoffatomen verbunden mit dem Sauerstoff durch ein gesättigtes Kohlenstoffatom oder Carbonyl;
Z ist eine Funktionalität zur Verknüpfung von X mit dem Sauerstoff an der 2-Position;
X ist ein Enzymdonorfragment aus β-Galactosidase aus 27 bis 60 Aminosäuren; und
n ist 1 oder 2.

11. Kit, umfassend eine Verbindung nach Anspruch 10, Enzymakzeptor für den genannten Enzymdonor und einem gekürzten extrazellulären Teil eines IP₃R mit mindestens der etwa 200-fachen Affinität für IP₃ als das intakte IP₃R.

## Revendications

1. Procédé d'essai de liaison de protéine pour doser du D-myo-inositol,1,4,5 triphosphate (IP₃) dans un échantillon en employant en tant que réactifs un conjugué d'IP₃ et d'un fragment d'enzyme pour la complémentation en enzyme en tant que marqueur détectable joint par une liaison ou un espaceur à la position 2-hydroxyle, et une protéine de liaison qui est une partie extracellulaire tronquée d'un récepteur d'IP₃ (IP₃R) ayant au moins 200 fois l'affinité pour l'IP₃ que l'IP₃R intact, ledit procédé comprenant :
la combinaison, dans un milieu d'essai, dudit échantillon, dudit conjugué et de ladite protéine de liaison, et l'incubation dudit mélange pendant un temps suffisant pour que tout IP₃ et ledit conjugué se lient à ladite protéine de liaison ; et
la détection du marqueur lié ou non lié en tant que dosage de l'IP₃ présent dans l'échantillon.

2. Essai de liaison de protéine selon la revendication 1, ledit essai étant dans un format homogène.

3. Essai de liaison de protéine selon la revendication 1, dans lequel ledit échantillon est un lysat cellulaire et dans lequel ledit lysat cellulaire a été traité pour bloquer les kinases et les phosphatases et préparer ledit échantillon pour ledit essai.

4. Essai de liaison de protéine selon la revendication 1, dans lequel ladite protéine de liaison n'a pas plus d'environ 600 acides aminés et comprend au moins les acides aminés 226 à 578 de l'IP₃R de souris de type 1.

5. Essai de liaison de protéine selon la revendication 1, dans lequel ladite protéine de liaison est une protéine de fusion de jusqu'à 1,5 k acides aminés.

6. Procédé selon la revendication 1, dans lequel l'ordre d'addition des réactifs est : (a) combinaison dudit échantillon avec ladite protéine de liaison ; et (b) addition dudit conjugué, en incubant après (a) et (b).

7. Procédé d'essai de liaison de protéine pour doser l'IP₃ dans un échantillon en utilisant un format homogène, en employant en tant que réactifs un conjugué d'IP₃ et d'un donneur d'enzyme (ED) de 37 à 60 acides aminés dérivé de β-galactosidase joint par un espaceur à la position 2-hydroxyle, et une partie extracellulaire tronquée d'un IP₃R ayant au moins environ 200 fois l'affinité pour l'IP₃ que l'IP₃R intact, ledit procédé comprenant :
la combinaison, dans un milieu d'essai, des constituants dans l'ordre suivant : ledit échantillon, ladite protéine de liaison, ledit conjugué et un accepteur d'enzyme (EA), et l'incubation, après chaque combinaison, pendant un temps suffisant pour la formation d'un complexe entre lesdits constituants d'essai ;
l'addition du substrat pour ladite β-galactosidase ; et
la détection du renouvellement de ladite β-galactosidase dudit substrat en tant que dosage de l'IP₃ présent dans l'échantillon.

8. Procédé d'essai de liaison de protéine pour doser l'IP₃ dans un échantillon en employant en tant que réactifs un conjugué d'IP₃ et d'un fragment d'enzyme pour la complémentation en enzyme en tant que marqueur détectable joint par une liaison ou un espaceur à la position 2-hydroxyle, et une partie extracellulaire tronquée d'un IP₃R ayant au moins environ 200 fois l'affinité pour l'IP₃ que l'IP₃R intact, ledit procédé comprenant :
la combinaison, dans un milieu d'essai, dudit échantillon, dudit conjugué, de ladite protéine de liaison et d'un réducteur chimique, et l'incubation dudit mélange pendant un temps suffisant pour que tout IP₃ et ledit conjugué se lient à ladite protéine de liaison ; et
la détection du marqueur lié ou non lié en tant que dosage de l'IP₃ présent dans l'échantillon.

9. Essai de liaison de protéine selon la revendication 8, dans lequel ledit réducteur chimique est un thiol.

10. Composé de formule : 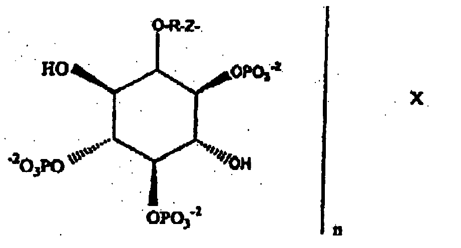 dans laquelle :
R est un groupe de liaison neutre de 4 à 20 atomes de carbone lié à l'oxygène par un atome de carbone saturé ou un carbonyle ;
Z est une fonctionnalité pour lier X à l'oxygène en position 2 ;
X est un fragment donneur d'enzyme de la β-galactosidase de 27 à 60 acides aminés ; et
n est 1 ou 2.

11. Trousse comprenant un composé selon la revendication 10, un accepteur d'enzyme pour ledit donneur d'enzyme et une partie extracellulaire tronquée d'un IP₃R ayant au moins environ 200 fois l'affinité pour l'IP₃ que l'IP₃R intact.
